Europäisches Patentamt

European Patent Office

Office européen des brevets

(19)

(11) Veröffentlichungsnummer: **0 001 274**
**B1**

(12) **EUROPÄISCHE PATENTSCHRIFT**

(21) Anmeldenummer: **78100915.4**

(22) Anmeldetag: **18.09.78**

(51) Int. Cl.³: **C 07 C 89/00,**
**C 07 C 85/11,**
**C 07 C 91/10, //**
**C 07 C 79/18**

(54) **Verfahren zur Herstellung von Serinol**

(30) Priorität: **21.09.77 DE 2742981**

(43) Veröffentlichungstag der Anmeldung:
**04.04.79 Patentblatt 79/07**

(45) Bekanntmachung des Hinweises auf die Patenterteilung:
**20.08.80 Patentblatt 80/17**

(84) Benannte Vertragsstaaten:
**BE CH DE FR GB NL SE**

(56) Entgegenhaltungen:
**Chemisches Zentralblatt**
**90. Jahrgang, Band III, 1919, Berlin**
**E. SCHMIDT et al "Über einige Derivate des Tri-**
**methylenglykols" Seite 125 bis 126**

(73) Patentinhaber: **Schering Aktiengesellschaft Berlin**
**und Bergkamen**
**Mullerstrasse 170**
**178 Postfach 65 0311**
**D - 1000 Berlin 65 (DE)**

(72) Erfinder: **Pfeiffer, Heinrich, Dr.**
**Pulfrichzeile 9**
**D - 1000 Berlin 20 (DE)**

Courier Press, Leamington Spa, England.

### Verfahren zur Herstellung von Serinol

Die Erfindung betrifft ein Verfahren zur Herstellung von 1,3-Dihydroxy-2-amino-propan, dadurch gekennzeichnet, dass man das Natriumsalz von 1,3-Dihydroxy-2-nitro-propan in einem inerten Lösungsmittel in Gegenwart eines Hydrierungskatalysators hydriert.

1,3-Dihydroxy-2-amino-propan, das in der Literatur auch als Serinol bezeichnet wird, ist eine kleine physiologisch gut verträgliche Base, die als Zwischenprodukt zur Herstellung von sehr gut verträglichen Röntgenkontrastmitteln zunehmend Beachtung findet. Beispielsweise seien das Dt-Patent 2 547 789 und die deutsche Patentanmeldung P 26 28 517.6 genannt, in denen die günstigen Eigenschaften von Serinol enthaltenden Röntgenkontrastmitteln und Verfahren zu ihrer Herstellung beschrieben werden.

Bisher sind aus der Literatur für die Herstellung von Serinol nur äusserst aufwendige Mehrstufensynthesen bekannt die technisch nicht verwertbar sind und schlechte Ausbeuten liefern.

So konnten D. Piloty und O. Ruff (Ber. dtsch. Chem. Ges. *30*, 2061 (1897) ausgehend von Dihydroxyaceton nach Herstellung des Dihydroxyacetonoxims und anschliessender Reduktion mit Na-Amalgam das Serinoloxalat oder Hydrochlorid in 15%iger Ausbeute isolieren.

Ein weiterer Syntheseweg wird von E. Schmidt und R. Wilkendorf beschrieben (Ber. dtsch. Chem. Ges. 52, 389 (1919)). Die Autoren kondensieren Paraformaldehyd und Nitromethan in Gegenwart wässriger Kalilauge und überführen das Reaktionsprodukt ohne Isolierung der Zwischenprodukte in das Natrium-Salz von 1,3-Dihydroxy-2-nitro-propan. Das Natrium-Salz, das als Ausgangsprodukt für die folgenden Reaktionsstufen dient, fällt in 91%iger Ausbeute mit 2 Mol Methanol an. Um die Nitro-Gruppe in die Amino-Gruppe überführen zu können, ist es nach den Erfahrungen der Autoren erforderlich, vor der Hydrierung die freie Nitro-Verbindung herzustellen. Die Umwandlung des Natrium-Salzes einer Nitro-Verbindung in die freie Nitro-Verbindung wird jedoch ganz allgemein von Nebenreaktionen begleitet, die auch zur Hauptreaktion werden können.

Nach Houben-Weyl, Bd. X/1 S. 456 (1969) gelingt die Darstellung des freien Nitro-propandiols mit Mineralsäuren nicht bzw. nur in ganz geringer Menge. Schmidt und Wilkendorf setzen das Na-Salz mit Salicylsäure in Äther um und isolieren das noch nicht völlig reine 1,3-Dihydroxy-2-nitro-propan in 59%iger Ausbeute. Die anschliessende katalytische Hydrierung gelingt nach den Erfahrungen der Autoren mit einem Pd-Barium-sulfat-Katalysator am besten in oxalsaurer Lösung. In neutraler Lösung verläuft sie sehr langsam und in alkalischer oder mineralsaurer Lösung unterbleibt sie.

Aus der oxalsauren Lösung wird das neutrale Oxalat von 1,3-Dihydroxy-2-amino-propan in 93%iger Ausbeute isoliert. Daraus wird schliesslich das Serinol als viskose Flüssigkeit durch Fällung von Bariumoxalat mittels Bariumhydroxidlösung ohne Angabe einer Ausbeute freigesetzt.

Der Erfindung liegt die Aufgabe zugrunde, eine verbessertes wirtschaftlich verwertbares Verfahren zur Herstellung von Serinol zu erreichen. Es wurde überraschenderweise gefunden, dass anstelle des nur schwierig herstellbaren freien 2-Nitro-propan-1,3-diols das technisch gut zugängliche reine Natriumsalz unter geeigneten Bedingungen zum Serinol hydriert werden kann. Die Verbesserung liegt einesteils in der technisch einfachen Herstellung von Serinol in einem Einstufenverfahren anderenteils darin, dass durch Unterdrükkung von Nebenreaktionen in Ausbeuten bis zu 82% ein Produkt von sehr hohem Reinheitsgrad erhalten wird, das erstmals in Kristallform vorliegt.

Das erfindungsgemässe Verfahren ist im Gegensatz zu dem oben beschriebenen Dreistufenverfahren ein Einstufenverfahren, bei dem in Gegenwart eines üblichen Hydrierungskatalysators das Natriumsalz des 1,3-Dihydroxy-2-nitro-propans, das 2 Mol Wasser oder 2 Mol Methanol enthalten kann, zum Serinol hydriert wird.

Zur Neutralisation der während der Hydrierung entstehenden Natronlauge kann dem Reaktionsgemisch eine gepufferte Säure wie beispielsweise Ammoniumchlorid, Ammoniumsulfat, Ammoniumphosphat u.a. zugesetzt werden. Die Na-Salze dieser Säuren sollen bei der Aufarbeitung der Hydrieransätze gut abtrennbar sein. Die Hydrierung verläuft jedoch ähnlich gut ohne Zusatz eines zur Neutralisation der Natronlauge geeigneten Stoffes oder mit unterschüssiger Menge. In jedem Falle ist es jedoch notwendig, vor Destillation des Reaktionsgemisches die restliche Natronlauge zu neutralisieren.

Die Hydrierung wird unter Verwendung üblicher metallhaltiger Hydrierungskatalysatoren durchgeführt.

Als Hydrierungskatalysatoren seien beispielsweise genannt: nickel-, palladium-, platinoder rhodiumhaltige katalysatoren wie beispielsweise Pd-Bariumsulfat, Pt-IV-oxid, Rd-Kohle und bevorzugt Raney-Nickel oder Nickel-Mischkontakte.

Als Reaktionsmedium sind alle bei Hydrierungen gebräuchlichen inerten Lösungsmittel geeignet, sofern sie das Serinol lösen, zum Beispiel niedere Alkohole wie Methanol, Äthanol, Isopropanol und Wasser und deren Gemische. Da es bei Hydrierungen nicht günstig ist, hochkonzentrierte lösungen einzusetzen, verwendet man vorteilhafterweise 5—25%ige Na-Salz-Lösungen.

Die Hydrierung kann sowohl bei Raumtemperatur als auch bei niedrigerer oder erhöhter Temperatur durchgeführt werden. Für die Durchführung der Reaktion, die exotherm abläuft, wird vorzugsweise eine Temperatur zwischen 0° und 80° gewählt.

Die Hydrierung kann sowohl bei Normaldruck als auch bei erhöhtem Druck erfolgen, wobei ein Wasserstoffdruck von 1—100 atm. als günstig anzusehen ist.

Nach der Hydrierung wird die Reaktionsmischung gegebenenfalls unter Zusatz eines Neutralisationsmittels in üblicher Weise aufgearbeitet. Beispielsweise kann das Lösungsmittel abdestilliert und das entstandene anorganische Salze ausgefällt und abgetrennt werden. Das rohe Serinol kann gegebenenfalls durch Destillation gereinigt werden.

Herstellung der Ausgangsverbindung.

Eine Lösung von 48,8 g (0,8 Mol) Nitromethan in 750 ccm Methanol wird mit 83 g Paraformaldehyd (2,76 Mol) versetzt. Nach Zusatz von 15 Tropfen 33%iger wässriger KOH wird das Reaktionsgemisch unter Rühren zum Sieden erhitzt. Zur klaren Lösung tropft man unter Eiskühlung und Rühren eine Lösung von 54 g Natriummethylat (1,0 Mol) in 350 ccm Methanol. Das Natrium-Nitropropandiol kristallisiert mit 2 Mol Methanol aus.
Ausbeute: 151 g = 91% der Theorie.

200 g der rohen Verbindung werden in 1 Liter Wasser gelöst. Die dunkelbraune Lösung wird bis zum Brei eingeengt. Der auskristallisierte Niederschlag wird abgesaugt und mit sehr wenig Wasser nachgewaschen. Nach Trocknen bei 30°C (im Vakuum) werden 138,7 g (76,7%) de Natriumsalzes des 1,3-Dihydroxy-2-nitro-propan als Dihydrat erhalten.
Schmelzpunkt: 110—112°C (unter Zersetzung).

Beispiel 1
1,3-Dihydroxy-2-amino-propan; Serinol.

71,6 g (400 mMol) Na-nitro-propandioldihydrat werden in 900 ml Methanol suspendiert. Nach Einfüllen in den Druckautoklaven werden dem Ansatz 17,1 g (320 mMol) Ammoniumchlorid sowie 8 g Raney-Nickel zugesetzt. Die Hydrierung wird bei 70 atm Wasserstoff und Raumtemperatur begonnen. Die Wasserstoffaufnahme ist in wenigen Stunden beendet. Anschliessend wird der katalysator abgetrennt und die Lösung mit 4,3 g (80 mMol) Ammoniumchlorid versetzt. Das methanol wird im Vakuum abdestilliert und der ölige Rückstand mit 80 ml Isopropanol auf eine Fritte gespült. Das Kochsalz wird abgesaugt und verworfen. Aus dem Filtrat wird Isopropanol in Vakuum abdestilliert. Das Serinol destilliert im Vakuum bei $Kp_7$ 136—138°C.
Ausbeute: 27,5 g = 75,5%. Reinheit aufgrund der Gaschromatographie 99,6%ig. Nach längerem Stehen oder beim Animpfen kristallisiert das Serinol;
Schmelzpunkt ca. 50°C.

Analog Beispiel 1 wurde eine Reihe ähnlicher Versuche durchgeführt, um die Variationsbreite des Verfahrens zu belegen.

Die folgende Tabelle fasst die Ergebnisse zusammen:

## TABELLE

Hydrierungsversuche

Eingesetzt jeweils  $0,4 \text{ Mol Na-Salz} \times 2\,H_2O \text{ (I)} \cong 71,6 \text{ g}$

oder  $0,4 \text{ Mol Na-Salz} \times 2 \cdot \text{MeOH (II)} \cong 82,8 \text{ g}$

| Beispiel Nr. | Na-Salz | Lösungs-m. | | Neutralisations-m. | | $H_2$-Anf.-Druck atm | Max. Temp. °C | Ausb. n. Dest, % | Reinheit n. G C % |
|---|---|---|---|---|---|---|---|---|---|
| | | Art | Menge ml | Art | % a | | | | |
| 1 | I | MeOH | 900 | $NH_4Cl$ | 80 | 70 | 27 | 75,5 | 99,6 |
| 2 | I | i-PrOH | 900 | $NH_4Cl$ | 95 | 70 | 27 | 64,8 | 99,9 |
| 3 | I | $H_2O$ | 900 | $(NH_4)_2SO_4$ | 95 | 70 | 27 | 63,7 | 99,6 |
| 4 | I | EtOH | 900 | $NH_4Cl$ | 80 | 70 | 27 | 70,2 | 98,0 |
| 5 | I | MeOH | 900 | $NH_4Cl$ | 0 | 70 | 27 | 71,9 | 99,9 |
| 6 | I | MeOH | 900 | $NH_4Cl$ | 100 | 25 | 10 | 82,0 | 99,9 |
| 7 | II | MeOH | 900 | $NH_4Cl$ | 80 | 70 | 27 | 73,0 | 98,7 |
| 8 | I | MeOH | 450 | $NH_4Cl$ | 80 | 7 | 44 | 77,7 | 99,4 |

a = %-Satz des vor der Hydrierung zugesetzten Neutralisationsmittels.

### Beispiel 9

In einem 1-Liter-Autoklaven mit magnetischer Mischvorrichtung wird eine Suspension von 41,4 g = 0,2 Mol Natrium-nitro-propandiol-1,3 mit rund 2 Mol Kristallmethanol in 450 ml Methanol mit 8,6 g = 0,16 Mol Ammoniumchlorid und 4 g eines 10%igen Palladium-Kohle-Katalysators bei einem Angangsdruck von 70 at Wasserstoff und bei Raumtemperatur hydriert. Nach wenigen Stunden kommt die Wasserstoffaufnahme bei 32 at zum Stillstand. Die Aufarbeitung gemäss Beispiel 1 unter Zusatz von 2,15 g Ammoniumchlorid liefert bei der Destillation 13,1 g = 71,5% der Theorie Serinol; $Kp_6$ 134—137°C, Reinheit nach Gaschromatographie 98,5%.

### Patentanspruch

Verfahren zur Herstellung von 1,3-Dihydroxy-2-amino-propan, dadurch gekennzeichnet, dass man das Natriumsalz von 1,3-Dihydroxy-2-nitro-propan in einem inerten Lösungsmittel in Gegenwart eines Hydrierungskatalysators hydriert.

### Claim

Process for the preparation of 1,3-dihydroxy-2-amino-propane, characterised in that the sodium salt of 1,3-dihydroxy-2-nitropropane is hydrogenated in an inert solvent in the presence of a hydrogenating catalyst.

### Revendication

Procede de préparation du dihydroxy-1,3-amino-2-propane, caractérisé en ce qu'on hydrogène le sel sodique du dihydroxy-1,3-nitro-2-propane dans un solvant inerte en présence d'un catalyseur d'hydrogénation.